# EUROPEAN PATENT APPLICATION

(11) **EP 4 618 052 A1**
(43) Date of publication of application: **17.09.2025**
(21) Application number: 23813900.0
(22) Date of filing: 27.10.2023
(51) Int. Cl.: G07F 9/02, G07F 11/00, G07F 11/62, G16H 20/13, G07F 17/00

(54) **ELECTRONIC DISPENSING DEVICE FOR STORING, PACKAGING AND DISTRIBUTING AT LEAST ONE PRODUCT IN FLOWPACK-TYPE PACKAGING**

(30) Priority: 11.11.2022 PT 2022118340
(71) Applicant: Raclac, S.A., 4770-170 Vila Nova de Famalicão (PT)
(72) Inventor: COSTA, Pedro Miguel, 4770-170 Vila Nova de Famalicão (PT)
(74) Representative: Monteiro Alves, Inês
(86) International application number: PCT/PT2023/050040
(87) International publication number: WO 2024/102012

(57) **Abstract**

The present invention describes an electronic dispensing device for storing, packaging and distributing at least one product in flowpack packaging (15), wherein said device presents constructive characteristics that enable the packaging to remain protected from contamination by outside contamination and moisture.

The packaging is organized in an orderly manner inside the dispensing device, so as to facilitate and streamline the collection by a user, in addition to the advantages of eliminating cross-contamination.

The electronic dispensing device of the invention further comprises a computer processing system, this being a crucial tool for managing the use and consumption of flowpack packaging (15) and products contained therein.

## Description

### Technical Domain

The present invention falls into the field of recipients for packaging or transporting articles or materials and refers to an electronic dispensing device for storing, packaging and distributing at least one product in flowpack packaging.

### Prior Art

Flowpack packaging appears in diverse market segments and corresponds to a type of flexible packaging that envelops a merchandise entirely, such that the upper and lower ends are wrapped and sealed.

Besides improved protection for the product and extending the useful life thereof, flowpack packaging is versatile and easy to handle, enabling wrapping of foodstuffs - like cookies and biscuits - to soaps, electronic products and disposable use gloves.

However, for flowpack packaging to remain free of outside contamination and not to come into continuous contact (or have minimized contact) with surrounding contamination (such as particles, dirt, pathogens, and others) or moisture, it is of the utmost importance that they be adequately stored and packaged, and that the product can be collected with minimal or zero cross-contamination when they are used.

In general, dispensing devices of the state of the art, such as the one described in US10579958B2, comprise various compartments or channels inside of which one or more products can be stored in "stacked" formation. Said compartments or channels may have a visualization area through which the user may visually observe the storage of the items to be purchased.

Further, as shown in document US9779574B1, the dispensing device can be driven by the user, who presses a button to move the pusher related to the product of interest. Accordingly, the pusher comes into direct contact with the product to be purchased and pushes it off the storage shelf, making it drop into a release tray, from where the user may withdraw it.

With the aim of providing a dispensing device that eliminates any risk of cross-contamination and cumulative contamination of the environment surrounding the packaging and product on the inside, the present invention refers to an electronic dispensing device for storing, packaging and distributing at least one product in flowpack packaging.

In a preferred embodiment of the present invention, the flowpack packaging is packaging for sterile or non-sterile, single-use gloves which need to be stored, from the production up to use thereof, in an environment free of outside contamination. Said flowpack packaging consists of a casing with an opening, from which the gloves are collected when they need to be worn. In this packaging, the gloves are positioned with the wrist facing towards the opening, which minimizes the risks of cross-contamination by the user.

### Brief description of the invention

The present invention refers to an electronic dispensing device for storing, packaging and distributing at least one product in flowpack packaging (15) comprising:
a packaging zone (13) comprising:
an insertion zone (1) of at least one flowpack packaging, wherein said insertion zone (1) is adapted for inserting at least one flowpack packaging and is closed by means of a door of the insertion zone (10);
a collection zone (2) of the said at least one flowpack packaging, wherein said collection zone (2) is adapted so that a user has access to at least one product contained in the at least one flowpack packaging and is closed by means of a door of the collection zone (11), wherein the collection zone (2) is disposed beneath the insertion zone (1); and
a visualization zone (3) of the at least one flowpack packaging packaged in the insertion zone (1), comprising at least one display screen (5), wherein the visualization zone (3) is disposed between the door of the insertion zone (10) and the door of the collection zone (11);
   and
a computer processing system adapted for communicating with an electronic database and with a display screen (5), so as to record all events related to the use and inventory of flowpack packaging (15) and products contained therein, packaged in said electronic dispensing device;
wherein the packaging zone (13) comprises at least one guide (8), wherein the guide (8) is disposed inside said packaging zone (13) and forms a plane which extends from the insertion zone (1) to the collection zone (2), said guide (8) further comprising at least one elastic means (9) which connects to the front face of the packaging zone (13); and
wherein the computer processing system is housed in an electronics zone (14), wherein said electronics zone (14) is disposed beneath the collection zone (11) and is separated from the packaging zone (13) by a boundary wall.

### Technical Problem

One of the main problems noted in the state of the art refers to the possibility of contamination of the packaging and product on the inside by the surrounding environment inside the storage, packaging and distribution device itself, either by atmospheric contamination (such as particles, dirt, pathogens, and others) or moisture.

Another problem inherent to the state of the art relates to the fact that the existing dispensing devices do not cumulatively enable access to the product inside the flowpack packaging without preventing cross-contamination between the user and the dispenser, that is to say, a dispensing device that keeps the product free of the cross-contamination, but which permits access to the packaging and to the product without the need to touch the dispensing device.

Another problem noted in the state of the art lies in the fact that there is no dispensing device that comprises a computer processing system adapted for communicating with an electronic database and with a display screen, so as to enable the management of the consumption and the inventory of the said dispensing device, notably, the location of the dispensing device, the use data and inventory of the different products; and other management potentialities.

### Solution to the Problem

The present invention solves the problems of the state of the art by means of storage, packaging and distribution of products in flowpack packaging by way of a specially-adapted electronic dispensing device which enables access by a user in a practical, efficient and secure manner.

The collection zone of the packaging is accessed through the opening and the automatic closure of the respective door by activating a movement or presence sensor, whereby avoiding user-product cross-contamination potentially caused by the user touching the dispenser.

For example, for the cases where the flowpack packaging is packaging for sterile or non-sterile, single-use gloves, the present invention enables the product (gloves) to be kept inside the packaging continuously upright, enabling the user to access solely the wrist - accordingly, cross-contamination is avoided. Studies prove that the vertical and wrist disposition of the gloves reduce cross contamination and consequences thereof, by avoiding human touch in the zones of the palms of the hand or fingers of said gloves, which are the contact zones between patient and carer.

Prior to distribution, the packaging is housed in an environment sealed against surrounding outside contamination and is not subject to continuous contact with particles, dirt, pathogens and moisture. The electronic dispensing device further comprises a storage zone adapted to store at least one flowpack packaging under the same conditions.

The electronic dispensing device also comprises a computer processing system adapted for communicating with an electronic database and with a display screen, so as to record all events related to the use and inventory of flowpack packaging and products contained therein, packaged in the electronic dispensing device, so as to assist in managing the inventory and replenish the packaging / product. This system equally enables the consumption data to be analyzed per area of utilization, size or other characteristics of the product contained in the flowpack packaging, analysis of replenishment / re-stockage time, among many other management applicabilities.

### Advantageous Effects of the Invention

The electronic dispensing device of the present invention bears constructive characteristics that enable the flowpack packaging, and the product inside, to remain protected from contamination by external particles (such as particles, dirt, pathogens) and moisture.

The packaging is organized in an orderly manner inside the dispensing device, so as to facilitate and streamline the collection by a user, in addition to the advantages of eliminating cross-contamination. For cases where the flowpack packaging is packaging for sterile or non-sterile, single-use gloves, this is crucial for the product and packaging to perform its role: to protect the product from contamination with subsequent protection of the user, third parties or product from the consequences of cross-contamination. The electronic dispensing device of the present invention enables the collection of the gloves through the most correct and safest place - the wrist - thus avoiding cross-contamination with the palm and the fingers, which are sites of greater likelihood of contact and inherently more at risk of cross-contamination in a subsequent procedure.

The computer processing system of the dispensing device is a crucial tool in managing the use and inventory of the flowpack packaging and products contained therein, and products contained therein, providing for efficient and accurate management. Said computer processing system also enables greater streamlining of product distribution, as well as efficiency of human resources, since it allows distance monitoring of whether or not flowpack packaging needs to be replenished, in addition to controlling the quantities consumed, avoiding unnecessary trips to the sites of use and decreasing the deviation of materials / products contained inside the flowpack packaging.

### Brief Description of the Figures

With the purpose of providing an understanding of the principles according to the embodiments of the present invention, reference will be made to the embodiments illustrated in the figures and to the terminology used to describe them. In any case, it should be understood that there is no intention of limiting the scope of the present invention to the contents of the figures.

Any subsequent alterations or modifications of the inventive characteristics illustrated herein, as well as any additional applications of the principles and embodiments of the invention illustrated, which would normally occur to a person skilled in the art in possession of this specification, are considered as being within the scope of the invention claimed.
**Fig. 1** Illustrates an embodiment of the electronic dispensing device for storing, packing and distributing at least one product in flowpack packaging comprising storage zone, in a front view with the insertion, collection and storage zones displayed.
**Fig. 2** Illustrates an embodiment of the electronic dispensing device for storing, packing and distributing at least one product in flowpack packaging, and specifically the products contained inside, comprising storage zone, in a front view with those with the insertion, collection and storage zones displayed closed by the respective doors.
**Fig. 3** Illustrates an embodiment of the electronic dispensing device for storing, packing and distributing at least one product in flowpack packaging comprising storage zone and flowpack packaging disposed vertically, in a front view with the insertion, collection and storage zones displayed.
**Fig. 4** Illustrates an embodiment of the electronic dispensing device for storing, packing and distributing at least one product in flowpack packaging, in a side view.

### Description of the Embodiments

The present invention refers to an electronic dispensing device for storing, packing and distributing at least one product in flowpack packaging (15), wherein said electronic dispensing device comprises:
a packaging zone (13) comprising:
an insertion zone (1) of at least one flowpack packaging (15);
a collection zone (2) of the said at least one flowpack packaging (15), wherein the collection zone (2) is disposed beneath the insertion zone (1); and
a visualization zone (3) of the at least one flowpack packaging (15) packaged in the insertion zone (1), wherein said visualization zone (3) comprises at least one display screen (5) and is disposed between the door of the insertion zone (10) and the door of the collection zone (11); and
a computer processing system adapted for communicating with an electronic database and with the display screen (5), so as to record all events related to the use and inventory of flowpack packaging (15) and products contained therein, packaged in said electronic dispensing device,
wherein the computer processing system is housed in an electronics zone (14), wherein said electronics zone (14) is disposed beneath the collection zone (11) and is separated from the packaging zone (13) by a boundary wall.

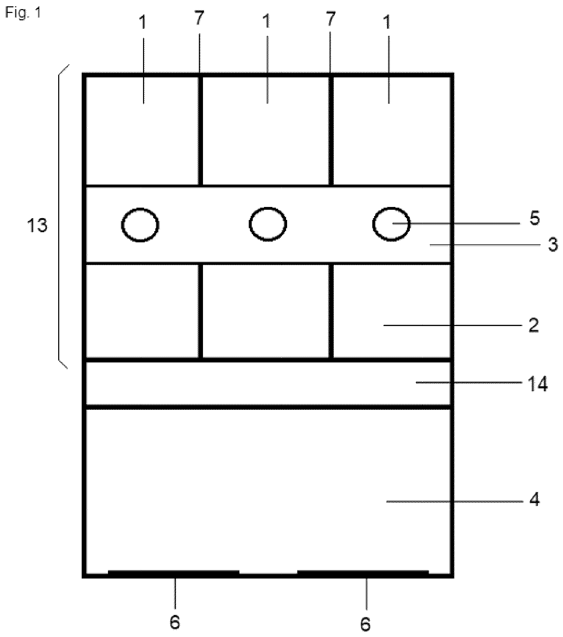

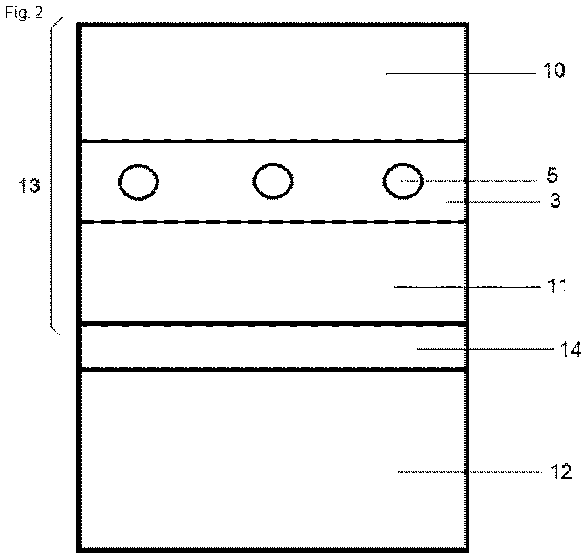

In a preferred embodiment of the present invention, the electronic dispensing device further comprises a storage zone (4) of at least one product in flowpack packaging (15), wherein said storage zone (4) is adapted for storing at least one flowpack packaging (15) and is closed by means of a door of the storage zone (12), wherein the storage zone (4) is disposed beneath the electronics zone (14). The electronic dispensing device of the present invention, comprising the storage zone (4) is represented in Figures 1 to 4 of the invention.

Said insertion zone (1) is adapted for inserting at least one product in flowpack packaging (15) and is closed by means of a door of the insertion zone (10).

Said collection zone (2) is adapted so that a user has access to at least one product contained in the at least one flowpack packaging (15) and is closed by means of a door of the collection zone (11).

The door of the collection zone (11) is opened by activating at least one movement or presence sensor (6), which is disposed in the front or side zone or in the base of the electronic dispensing device. In this sense, when approaching the sensor, the user activates the opening of the door of the collection zone (11).

Likewise, the closure of the door of the collection zone (11) is activated when the sensor is approached again. If the user forgets to activate the closure of the door or is prevented from so doing, the door of the collection zone (11) will automatically close at the end of a pre-set time interval. For matters of safety, the closure will be interrupted if excess force is detected on the door of the collection zone (11), indicating that it is still in use by the user.

Any movement or presence sensors known in the state of the art can be employed.

Said storage zone (4) is adapted for storing at least one flowpack packaging (15) and is closed by means of a door of the storage zone (12), wherein the storage zone (4) is disposed beneath the electronics zone (14).

The packaging zone (13) comprises at least one guide (8), wherein the guide (8) is disposed inside said packaging zone (13) and forms a plane which extends from the insertion zone (1) to the collection zone (2), said guide (8) further comprising at least one elastic means (9) which connects to the front face of the packaging zone (13).

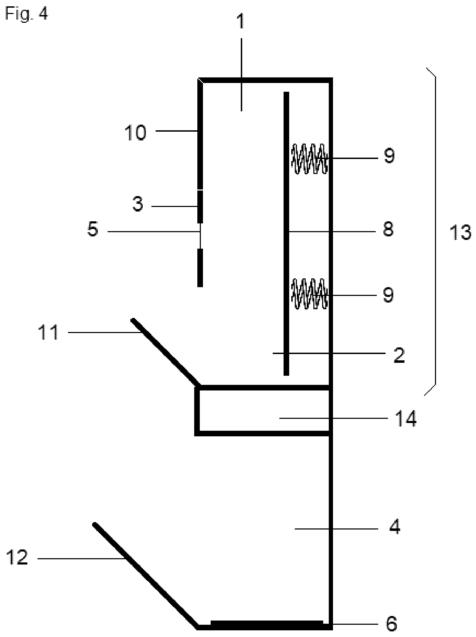

Said guide (8) further comprises at least one elastic means (9), which connects to the front face of the packaging zone (13). Accordingly, said at least one elastic means (9) pushes the at least one guide (8) against the front face of the packaging zone (13), keeping the flowpack packaging (15) in the desired position and orientation throughout the consumption thereof and preventing same from falling by action of gravity. In a preferred embodiment of the present invention, the elastic means (9) is selected from the group consisting of a spring or a rod made of elastomeric material. These components can be seen in Figure 4 of the present invention.

In an embodiment of the present invention, the electronic dispensing device comprises at least one partition (7), which extends from the insertion zone (1) to the collection zone (2) and which divides the packaging zone (13) into different adjacent compartments.

Each of the compartments can be configured to respectively house different flowpack packaging (15). In a preferred embodiment of the present invention, each of the different adjacent compartments comprises a visualization zone (3) with a corresponding display screen (5), adapted for indicating the type of product contained in each flowpack packaging (15) and for managing the inventory.

Figures 1, 2 and 3 of the present invention show the electronic dispensing device in a configuration that comprises two partitions (7), which delimit three adjacent compartments. However, the number of partitions and compartments may vary according to the product to be packaged and the consumer needs to be satisfied.

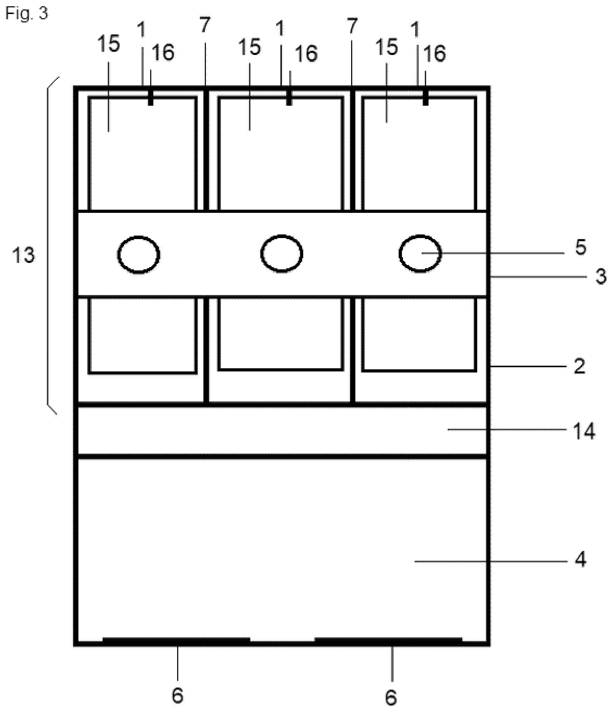

In a preferred embodiment of the present invention, showed in Figure 3, each of the different adjacent compartments comprises, in the upper part of the packaging zone (13), a fastening system (16), wherein said fastening system (16) can be selected from the group consisting of magnets, claws, pins, hooks or suction cups. The purpose of the fastening system (16) is to position and maintain the flowpack packaging (15) in vertical position, that is, parallel to the partitions (7) and to the guide (8), throughout the entire use thereof.

More specifically, the flowpack packaging (15) is introduced through the insertion zone (1) and suitably oriented to the proposed use (in a preferred embodiment, the upper part of the packaging is fastened in the upper part of the packaging zone (13) by means of the fastening system (16) and the lower part of the packaging, which comprises the opening for collecting the products, is positioned in the collection zone (2)). Accommodating said packaging (15) inside the packaging zone (13) occurs by way of the guide (8), which presses said packaging (15) against the front face of the electronic dispensing device, preventing it from falling by action of gravity, whereby maintaining its position throughout consumption. As the products are withdrawn by the collection zone (2), the elastic means decompresses (9) and the resulting distancing of the guide (8) relative to the front face of the device, so as to maintain suitable alignment of the packaging.

As indicated, the electronic dispensing device of the present invention further comprises a computer processing system adapted for communicating with an electronic database and with the display screen (5), so as to record all events related to the use and inventory of flowpack packaging (15) and of products contained therein, packaged therein.

As such, the electronic dispensing device of the present invention comprises a dedicated embedded software, configured to feed data (such as activating the opening of the collection zone, restocking and available inventory) and promoting the management of the use and the consumption of said flowpack packaging (15) and of products contained therein of the said device.

Communication is via Wi-Fi, Bluetooth or any other form of existing wireless communication or as may come to be developed for any pre-set period of time, by way of an application for computing equipment such as smartphones, tablets and computers.

For matters of security, the dispensing devices communicate with an electronic database (exclusive to the user in question), which is found stored in a temporary buffer. This electronic database is accessed by the main server over a pre-set time period, where all the information recorded therein will be retrieved and deleted. Thus, the data is not recorded locally.

In a preferred embodiment, the electronic dispensing device of the present invention further comprises a light indicator (not shown in the figures), which indicates the level of product available inside the flowpack packaging at any given time.

The electronic dispensing device of the present invention may be made of different materials, such as, but not limited to, wood or byproducts thereof, plastics, acrylics or others synthetic or natural polymer materials, recycled, recyclable, or non- recycled and non-recyclable.

In order to avoid any outside contamination, all the doors have a sealing mechanism and may or may not contain anti-bacterial finishing. Further, the electronic dispensing device may also be fastened to a wall or support.

The subject matter described above is provided as an illustration of the present invention and, therefore, cannot be interpreted in a way that limits it. The terminology used herein with the purpose of describing preferred embodiments of the present invention, must not be interpreted to limit the invention to the same.

As used in the specification, the definite and indefinite articles, in their singular form, are intended to be interpreted as also including the plural forms, unless the context of the description explicitly indicates otherwise.

The indefinite article "a" or "one" should be interpreted generally as "one or more", unless the sense of a singular embodiment is clearly defined in a specific situation.

It will be understood that the expressions "comprise" and "include", when used in this description, specify the presence of the characteristics, the elements, the components, the steps, and the related operations, however, they of the not exclude the possibility of other characteristics, elements, components, steps, and operations also being contemplated.

As used throughout this patent application, the expression "or" is used in the inclusive sense instead of the exclusive sense, unless the exclusive sense is clearly defined in a specific situation. In this context, a sentence of the type "X uses A or B" should be interpreted as including all the pertinent inclusive combinations, for example "X uses A", "X uses B" and "X uses A and B".

All the alterations, providing that they of the not modify the essential characteristics of the claims that follow, should be considered as being within the scope of protection of the present invention

### List of Reference Indications

1. Insertion zone
2. Collection opening
3. Visualization zone
4. Storage zone
5. Display screen
6. Movement or presence sensor
7. Partition
8. Guide
9. Elastic means
10. Door of the insertion zone
11. Door of the collection opening
12. Door of the storage zone
13. Packaging zone
14. Electronics zone
15. Flowpack packaging
16. Fastening system

### List of Citations

### List of citations follows:

### Patent Literature

patcit1: US10579958B2
patcit2: US9779574B1

## Claims

1. An electronic dispensing device for storing, packing and distributing at least one product in flowpack packaging (15) **characterized by** comprising:
a packaging zone (13) comprising:
an insertion zone (1) of at least one flowpack packaging (15), wherein said insertion zone (1) is adapted for inserting at least one flowpack packaging (15) and is closed by means of a door of the insertion zone (10);
a collection zone (2) of said at least one flowpack packaging (15), wherein said collection zone (2) is adapted so that a user has access to at least one product contained in the at least one flowpack packaging (15) and is closed by means of a door of the collection zone (11), wherein the collection zone (2) is disposed beneath the insertion zone (1); and
a visualization zone (3) of the at least one flowpack packaging (15) packaged in the insertion zone (1), comprising at least one display screen (5), wherein the visualization zone (3) is disposed between the door of the insertion zone (10) and the door of the collection zone (11);
and
a computer processing system adapted for communicating with an electronic database and with a display screen (5), so as to record all events related to the use and inventory of flowpack packaging (15) and products contained therein, packaged in said electronic dispensing device;
wherein the packaging zone (13) comprises at least one guide (8), wherein the guide (8) is disposed inside said packaging zone (13) and forms a plane which extends from the insertion zone (1) to the collection zone (2), said guide (8) further comprising at least one elastic means (9) which connects to the front face of the packaging zone (13); and
wherein the computer processing system is housed in an electronics zone (14), wherein said electronics zone (14) is disposed beneath the collection zone (11) and is separated from the packaging zone (13) by a boundary wall.

2. The electronic dispensing device for storing, packing and distributing at least one product in flowpack packaging (15), according to the preceding claim, **characterized by** comprising a storage zone (4) of at least one flowpack packaging (15), wherein said storage zone (4) is adapted for storing at least one flowpack packaging (15) and is closed by means of a door of the storage zone (12), wherein the storage zone (4) is disposed beneath the electronics zone (14).

3. The electronic dispensing device for storing, packing and distributing at least one product in flowpack packaging (15), according to any of the preceding claims, **characterized by** further comprising at least one movement or presence sensor (6) adapted for activating the opening and the closure of the door of the collection zone (11), wherein said at least one movement or presence sensor (6) is disposed in the front or side zone or in the base of the electronic dispensing device.

4. The electronic dispensing device for storing, packing and distributing at least one product in flowpack packaging (15), according to any of the preceding claims, **characterized by** comprising at least one partition (7), which extends from the insertion zone (1) to the collection zone (2) and which divides the packaging zone (13) into different adjacent compartments.

5. The electronic dispensing device for storing, packing and distributing at least one product in flowpack packaging (15), according to claim 4, **characterized in that** each of the different adjacent compartments comprises a visualization zone (3) with a corresponding display screen (5), adapted for indicating the type of product contained in each flowpack packaging (15) and managing the inventory.

6. The electronic dispensing device for storing, packing and distributing at least one product in flowpack packaging (15), according to any of claims 4 or 5, **characterized in that** each of the different adjacent compartments comprises, in the upper part of the packaging zone (13), a fastening system (16), wherein said fastening system (16) can be selected from the group consisting of magnets, claws, pins, hooks or suction cups.

7. The electronic dispensing device for storing, packing and distributing at least one product in flowpack packaging (15), according to any of claims 1 to 6, **characterized in that** the elastic means (9) is selected from the group consisting of a spring or a rod made of elastomeric material.

8. The electronic dispensing device for storing, packing and distributing at least one product in flowpack packaging (15), according to any of claims 1 to 7, **characterized in that** the computer processing system is configured to process data relative to managing the use and inventory of flowpack packaging (15) and products contained therein, packaged in said device and communicating said data through an application to computing equipment such as smartphones, tablets and computers.
